# FASCICULE DE BREVET EUROPEEN

(11) **EP 0 983 772 B1**
(45) Date de publication et mention de la délivrance du brevet: **01.10.2003**
(21) Numéro de dépôt: 99401891.9
(22) Date de dépôt: 26.07.1999
(51) Int. Cl.: A61M 16/12

(54) **Dispositif pour assistance respiratoire**
Vorrichtung zur Unterstützung der Atmungsfunktion
Respiratory assistance device

(30) Priorité: 03.09.1998 FR 9811027
(43) Date de publication de la demande: 08.03.2000
(73) Titulaire: Boussignac, Georges, 92160 Antony (FR)
(72) Inventeur: Boussignac, Georges, 92160 Antony (FR)
(74) Mandataire: Bonnetat, Christian

(56) Documents cités:
- EP-A- 0 390 684
- EP-A- 0 701 834
- WO-A-95/28193
- WO-A-97/18003
- US-A- 4 825 862

## Description

La présente invention a pour objet un dispositif pour assistance respiratoire, utilisable sur des patients dont la respiration spontanée est absente ou insuffisante, qu'ils soient placés ou non sous respiration artificielle.

On connaît divers dispositifs, tels que des masques, des sondes ou des canules orales, nasales, endotrachéales, trachéotomiques, destinés à faire la jonction entre un appareil de respiration artificielle et/ou d'anesthésie et le système respiratoire d'un patient. Ces dispositifs, essentiellement en forme de tubes, peuvent, selon le cas, comporter des moyens d'immobilisation tels que des pattes ou des collerettes au voisinage de l'extrémité proximale, pour le maintien sur la bouche ou le nez du patient, ou encore des ballonnets gonflables au voisinage de l'extrémité distale, pour le maintien par friction dans la trachée.

Les dispositifs connus présentent des inconvénients importants. Ainsi, par exemple, lorsqu'un tube de type connu est déconnecté du respirateur artificiel et que le patient a besoin d'air enrichi en oxygène, il est nécessaire d'introduire dans ledit tube une sonde reliée à une source d'oxygène. Par ailleurs, dans les cas de respiration spontanée insuffisante, le patient doit nécessairement rester relié au respirateur jusqu'au rétablissement complet de sa respiration spontanée.

Aussi, pour remédier à ces inconvénients, on a déjà proposé, par exemple dans les documents EP-A-0 390 684 et EP-A-0 701 834, des dispositifs d'assistance respiratoire qui, outre le canal principal formé par le tube, comportent au moins un canal auxiliaire, par exemple ménagé dans la paroi dudit tube, permettant l'injection d'un jet de gaz respirable (oxygène, air ou mélange air-oxygène) destiné à la ventilation du patient, ces canaux auxiliaires débouchant dans le canal principal au voisinage de l'extrémité distale de ce dernier.

Pour éviter que les jets de gaz respirable viennent frapper directement la muqueuse du patient sous ventilation, risquant par leur énergie cinétique de traumatiser ladite muqueuse, on prévoit dans ces derniers dispositifs qu'au moins l'extrémité distale desdits canaux auxiliaires débouchant dans le canal principal soit parallèle à celui-ci et que, en regard de l'orifice distal de chaque canal auxiliaire, soient prévus des moyens de déflexion desdits jets de gaz respirable de ventilation vers l'intérieur dudit canal principal.

Ainsi, les jets de gaz respirable traversant lesdits canaux auxiliaires sont défléchis vers l'axe du canal principal, lorsqu'ils pénètrent dans celui-ci. Des mesures expérimentales ont montré que, en aval desdits moyens de déflexion, il se forme, à l'intérieur dudit canal principal, une zone de pression de forme oblongue prenant naissance aux débouchés desdits canaux auxiliaires dans le canal principal et s'allongeant en direction axiale le long de l'axe dudit canal principal avec réduction progressive de sa section transversale, pour n'occuper que la partie centrale de celui-ci, mais que, en aval de ladite zone de pression élevée, la pression desdits jets de gaz respirable chute et les jets de gaz sortent à faible pression à travers l'orifice distal du tube. L'expérience a également montré, qu'en aval de la sortie distale du tube, la pression est faible et maintenue constante dans tout l'espace respiratoire. Cette pression est dépendante du débit de gaz respirable dans les canaux auxiliaires. Par suite, avec le dispositif d'assistance respiratoire conforme au document ci-dessus, on peut par exemple apporter de l'oxygène ou un mélange air-oxygène directement dans les poumons d'un patient, à hauteur de la carène, et supprimer ainsi l'espace mort qui existe dans les autres sondes connues et qui est d'environ un tiers du volume respiratoire total pour un adulte et environ la moitié pour les nouveau-nés prématurés. La suppression de cet espace mort correspond à une augmentation de performance du cycle respiratoire de plus de 25% dans tous les cas de malades et de près de 50% dans certains cas.

Les dispositifs des documents EP-A-0 390 684 et EP-A-0 701 834 sont donc particulièrement avantageuse. Cependant, il présente l'inconvénient de nécessiter une source de gaz respirable à pression élevée (plusieurs bars) pour alimenter lesdits canaux auxiliaires. Or, une telle source peut ne pas être disponible et il peut être intéressant, pour des fins de sécurité, de pouvoir utiliser des sources de gaz respirable à faible pression (par exemple inférieure à un bar).

Ainsi, l'objet de la présente invention est de perfectionner lesdispositifs des documents EP-A-0 390 684 et EP-A-0 701 834 pour pouvoir les faire fonctionner avec une source de gaz respirable à faible pression.

A cette fin, selon l'invention, le dispositif d'assistance respiratoire comportant un tube qui forme un canal principal et qui est destiné à être relié par son extrémité distale à une voie respiratoire d'un patient pour que ledit canal principal relie, à l'extérieur, le système respiratoire dudit patient, ledit dispositif comportant de plus au moins un canal auxiliaire qui est relié, à son extrémité proximale, à une source de gaz respirable pour pouvoir insuffler un jet d'un tel gaz respirable dans ledit système respiratoire et dont l'extrémité distale débouche dans ledit canal principal au voisinage de l'extrémité distale de ce dernier, des moyens de déflexion dudit jet de gaz respirable de ventilation en direction de l'intérieur dudit canal principal étant prévus en regard de l'orifice distal dudit canal auxiliaire, de sorte que, en aval desdits moyens de déflexion, il se forme, à l'intérieur dudit canal principal, une zone de pression de forme oblongue prenant naissance audit orifice distal et s'allongeant dans la direction distale le long de l'axe dudit canal principal avec réduction progressive de sa section transversale en s'écartant de la paroi interne dudit canal principal pour n'occuper que la partie centrale de ce dernier, est remarquable en ce qu'il comporte une bague disposée dans ledit canal principal en aval (par rapport audit jet de gaz respirable) desdits moyens de déflexion et en ce que ladite bague entoure ladite zone de pression oblongue en obturant au moins partiellement l'espace périphérique dudit canal principal compris entre ladite paroi interne de celui-ci et ladite zone de pression oblongue.

Le demandeur a trouvé que, grâce à cette bague, il pouvait utiliser une source de gaz respirable à plus faible pression tout en obtenant une zone de pression oblongue à pression identique, ou bien obtenir une zone de pression oblongue à pression plus élevée avec une source de gaz respirable à pression identique. Tout se passe donc comme si ladite bague, en restreignant périphériquement ledit canal principal et en ne laissant libre que la partie centrale de la section de celui-ci, permettait de mieux utiliser la pression de ladite source de gaz respirable pour la formation de ladite zone de pression oblongue.

Le demandeur a trouvé expérimentalement qu'il était avantageux que la distance séparant ladite bague desdits moyens de déflexion soit approximativement égale au diamètre de la partie distale dudit canal principal. Ladite bague pourrait être fixe à l'intérieur dudit canal principal ou même faire partie intégrante dudit tube. Cependant, il est préférable, afin de pouvoir optimiser le gain de pression sur la source de gaz respirable que cette distance puisse être réglable. A la même fin, il est également avantageux que le diamètre intérieur de ladite bague soit réglable, afin d'adapter au mieux l'orifice de cette dernière à la section centrale de ladite zone de pression. Il est alors intéressant de prévoir un jeu de bagues interchangeables, à diamètre intérieur différent, susceptibles d'être introduites et déplacées par coulissement à l'intérieur de l'extrémité distale dudit tube. En variante, il est possible d'utiliser des bagues réalisées sous la forme d'anneaux gonflables, de façon à pouvoir modifier aisément leur diamètre intérieur.

On remarquera de plus que, grâce à la présente invention, il est particulièrement aisé de prévoir un humidificateur dans la conduite reliant la source de gaz respirable au canal auxiliaire. En effet, la présente invention permet d'abaisser la pression du jet de gaz respirable dans ladite conduite à un niveau permettant une bonne humidification de celui-ci. On évite ainsi le dessèchement de la muqueuse du patient.

Lorsque le dispositif selon l'invention comporte avantageusement une pluralité de canaux auxiliaires, il est avantageux qu'au moins certains d'entre eux soient alimentés en commun en gaz respirable. Une telle alimentation en commun desdits canaux peut se faire par l'intermédiaire d'une bague de distribution, coaxiale audit tube. Par ailleurs, lesdits canaux auxiliaires non alimentés en commun peuvent servir à l'introduction de produits gazeux additionnels, tels que des produits médicamenteux.

Ainsi, on voit que le dispositif conforme à l'invention permet, en toute sécurité :
- l'humidification du gaz respirable insufflé,
- l'intubation de longue durée de l'assistance respiratoire sans assèchement,
- l'injection de médicaments ou d'anesthésiques pendant l'assistance respiratoire,
- la mesure dynamique des pressions, car il suffit de prévoir des canaux auxiliaires auxquels sont associées des sondes appropriées,
- l'établissement d'un microdébit de gaz respirable dans les canaux auxiliaires pour éviter l'obstruction desdits canaux par des mucosités,
- l'augmentation du volume échangé, car la pression est auto-limitée et il n'y a aucun risque d'écrasement des capillaires pulmonaires,
- la diminution pour une même quantité d'oxygène échangée de l'importance d'oxygène dans le mélange, ce qui diminue d'autant les effets secondaires de l'assistance,
- la possibilité d'utiliser des respirateurs moins coûteux que les respirateurs actuels.

Par ailleurs, il est avantageux, notamment pour des raisons de sécurité, que le dispositif conforme à la présente invention comporte une vanne commandable montée dans la conduite reliant la source de gaz respirable audit canal auxiliaire et que ladite vanne soit commandée par un capteur détectant l'expiration du patient. Ainsi, ladite vanne peut être fermée pendant l'expiration du patient, de sorte que celle-ci est libre à travers ledit tube, les jets de gaz respirable et ladite zone de pression oblongue centrale étant alors supprimés. De préférence, ledit capteur détecte l'expiration du patient en aval de ladite bague, c'est-à-dire du côté de celle-ci opposé auxdits moyens de déflexion.

Les figures du dessin annexé feront bien comprendre comment l'invention peut être réalisée. Sur ces figures, des références identiques désignent des éléments semblables.

La figure 1 est une vue schématique et partielle, en coupe axiale agrandie, d'un mode de réalisation du dispositif de l'invention.

Les figures 2 et 3 sont des coupes transversales, respectivement selon les lignes II-II et III-III de la figure 1.

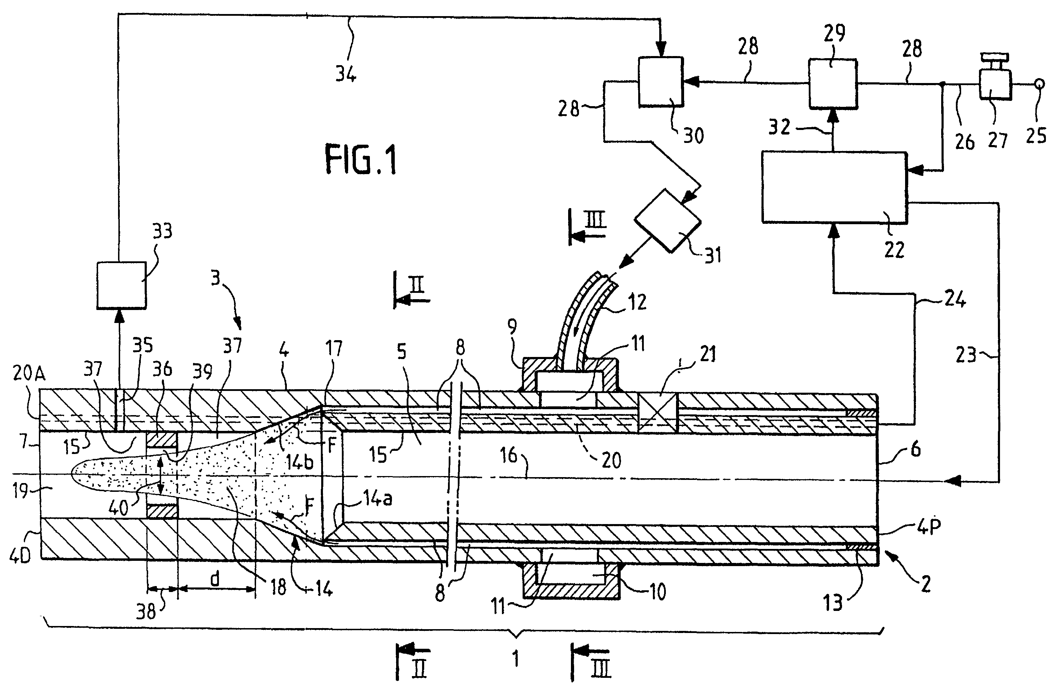

Sur la figure 1, on a représenté, schématiquement et à grande échelle, les seules extrémités proximale 2 et distale 3 d'un mode de réalisation 1 du dispositif selon l'invention. Ce mode de réalisation peut constituer, par exemple, une sonde endotrachéale oro-nasale avec ou sans ballonnet, une sonde endotrachéale pédiatrique, une sonde de monitorage des gaz, une sonde endobronchique, une sonde naso-pharyngée, une sonde d'intubation anatomique pour enfant, une sonde de Cole néonatale, une sonde canule de Gedel, une sonde nasale d'oxygénothérapie, un masque nasal ou bucconasal ou un ballon nasal pour traitement d'apnée du sommeil.

Le dispositif 1 comporte un tube 4, souple ou préformé (pour s'adapter à la morphologie du patient) délimitant un canal principal 5 débouchant, par l'orifice 6, à l'extrémité proximale 2 et, par l'orifice 7, à l'extrémité distale 3.

Ainsi, le canal principal 5 est capable d'assurer le passage entre les orifices 6 et 7, dont l'un (l'orifice 7) est destiné à se trouver à l'intérieur des voies respiratoires d'un patient, et l'autre (l'orifice 6) est destiné à se trouver à l'extérieur dudit patient. Cet orifice 6 peut déboucher à l'air libre et, dans ce cas, le patient peut inspirer de l'air frais et expirer l'air vicié à travers le canal principal 5. On peut également, comme cela est expliqué ci-après, relier l'orifice 6 à une source de gaz respirable sous pression et prévoir un système de valves unidirectionnelles, pour que le patient inspire le gaz respirable de ladite source à travers ledit canal principal 5 et expire le gaz vicié à l'air libre, également à travers ce canal principal.

Le diamètre du canal principal 5 est de l'ordre de quelques millimètres. Des essais satisfaisants ont été effectués avec des diamètres de 3 mm, 7 mm, 8 mm et 12 mm.

Par ailleurs, dans l'épaisseur de la paroi du tube 4, sont ménagés des canaux auxiliaires 8, s'étendant sur la presque totalité de la longueur du canal principal et destinés à être reliés à une source de gaz respirable sous pression, comme cela est décrit ci-après.

La liaison à la source de gaz respirable sous pression peut être réalisée au moyen d'une bague 9, entourant de façon étanche le tube 4, du côté de l'extrémité proximale 2, et délimitant une chambre annulaire étanche 10 autour dudit tube. Les canaux auxiliaires 8 sont mis en communication avec la chambre 10, grâce à des arrachements locaux 11 de la paroi du tube 4, et ladite chambre 10 est reliée à ladite source de gaz respirable par un conduit 12. Bien entendu, les extrémités proximales des canaux 8 sont obturées, par exemple par des bouchons 13, introduits à partir de la face d'extrémité proximale 4P du tube 4.

Les canaux auxiliaires 8 ont un diamètre plus petit que celui du canal principal 5. Le diamètre des canaux auxiliaires 8 est de préférence inférieur à 1 mm et, de façon avantageuse, il est de l'ordre de 400 à 800 microns. Du côté distal, les canaux auxiliaires 8 débouchent dans un évidement 14 de la paroi interne 15 du tube 4. L'évidement 14 est annulaire et centré sur l'axe 16 de l'extrémité distale 3. Il comporte une face 14a, sensiblement transversale ou légèrement inclinée de façon à constituer un évasement du canal principal 5, dans laquelle débouchent lesdits canaux auxiliaires 8 par leurs orifices 17, ainsi qu'une face 14b suivant la face 14a et convergeant en direction de l'axe 16.

Ainsi, lorsque les canaux auxiliaires 8 sont alimentés en gaz respirable sous pression à travers les éléments 9 à 12, les jets gazeux correspondants heurtent la face inclinée 14b, qui les défléchit en direction de l'axe 16 (flèches F sur la figure 1), engendrant à l'intérieur de l'extrémité distale 3 du canal principal 5 une zone de pression 18 de forme oblongue prenant naissance auxdits orifices distaux 17 et s'allongeant en direction de l'orifice distal 7 le long de l'axe 16 de ladite extrémité distale 3. La section transversale de la zone de pression 18 décroît progressivement depuis l'évidement 14 vers l'orifice distal 7, ladite zone de pression 18 s'écartant progressivement de la paroi interne 15 du tube 4 pour n'occuper que la partie centrale de l'extrémité distale 3 de ce dernier. En aval de la zone de pression 18, les jets de gaz respirable défléchis engendrent au voisinage de l'axe 16 une zone de dépression 19 favorisant la circulation gazeuse à l'intérieur du canal principal 5, de l'orifice proximal vers l'orifice distal. On favorise ainsi l'inspiration du patient.

Au moins un canal supplémentaire 20 est prévu dans l'épaisseur du tube 4 afin de déboucher en 20A dans la face d'extrémité distale 4D du tube 4 et servir de prise de pression.

A titre de sécurité, une soupape d'échappement tarée 21 peut être prévue dans l'extrémité proximale2 du tube 4. Ainsi, en cas de surpression accidentelle dans le canal principal 5, une fuite de gaz se produit à l'extérieur du patient, à travers la paroi du tube 4, pour éliminer instantanément cette surpression.

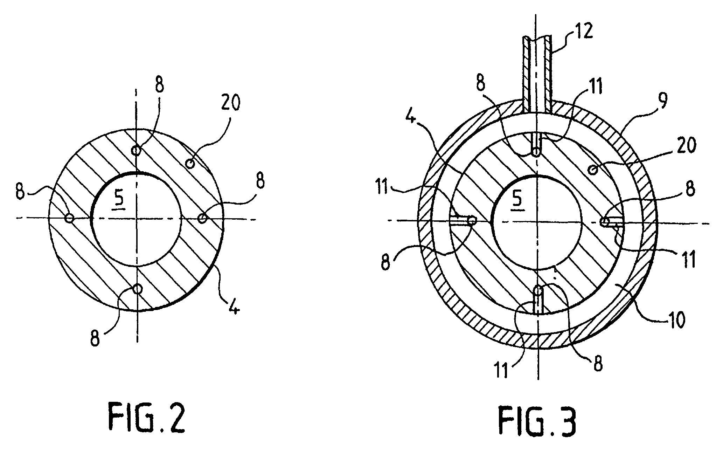

Comme le montrent les figures 2 et 3, les canaux auxiliaires 8 sont disposés régulièrement autour de l'axe du tube 4. Leur nombre est variable suivant les utilisations (adulte ou enfant), mais il est généralement compris entre trois et neuf. De plus, un au moins des canaux auxiliaires 8 peut être spécialisé pour apporter un fluide médical.

Le tube 4 du dispositif selon l'invention peut être réalisé en toute matière déjà utilisée dans les sondes respiratoires, par exemple en un chlorure de polyvinyle, avec un éventuel revêtement de silicone ou en acier permettant les injections à pression élevée.

Bien entendu, les dimensions du dispositif selon l'invention peuvent être très variables, essentiellement en fonction de la voie de mise en place du tube et de la taille du patient, qui peut être un adulte, un enfant, un nourrisson ou un prématuré.

Le dispositif 1 comporte de plus un dispositif d'alimentation et de commande 22, qui est respectivement relié à l'orifice 6 de l'extrémité proximale 2 du tube 4 par une liaison 23 et au canal supplémentaire 20 par une liaison 24.

Le dispositif d'alimentation et de commande 22 est alimenté en gaz respirable sous pression par une source 25, à laquelle il est relié par une conduite 26 sur laquelle est monté un détendeur-débitmètre réglable 27.

La sortie du détendeur-débitmètre 27 est reliée au conduit 12 par une conduite de dérivation 28 sur laquelle sont montés en série une première vanne commandable 29, une seconde vanne commandable 30 et un humidificateur 31.

La vanne commandable 29 est commandée par le dispositif d'alimentation et de commande 22 par l'intermédiaire d'une liaison 32.

La vanne commandable 30 est commandée par un détecteur 33 par l'intermédiaire d'une liaison 34. Le détecteur 33 est un capteur de pression ou un capteur de débit apte à détecter les passages de l'inspiration à l'expiration dans la respiration du patient. La prise de mesure 35 du détecteur 33 se trouve au voisinage de l'orifice distal 7.

Dans l'extrémité distale 3, entre ladite prise de mesure 35 et la face inclinée de déflexion 14b, est disposée une bague 36 entourant la zone de pression centrale 18 et occupant localement au moins en partie l'espace périphérique annulaire 37 compris entre ladite zone de pression centrale 18 et la paroi interne 15 de l'extrémité distale 3 du canal 5.

Grâce à une telle bague 36, comme cela a été expliqué ci-dessus, la pression de la source de gaz respirable 25, nécessaire à l'obtention de la zone de pression 18 peut être abaissée.

En règle générale, la distance d entre la bague 36 et la face inclinée de déflexion 14b est voisine du diamètre de la partie distale du canal principal 5.

Toutefois, il est avantageux, pour permettre d'obtenir la réduction optimale de pression nécessaire de la source 25, que cette distance d puisse être réglable, comme cela est illustré par la double flèche 38. Il est également avantageux, à la même fin, que le diamètre de l'ouverture centrale 39 de la bague 36 soit réglable, comme cela est illustré par la double flèche 40. Ce double réglage peut être obtenu grâce à un jeu de plusieurs bagues interchangeables 36 pouvant, au choix, être montées en coulissement dans l'extrémité distale du canal principal 5. La bague 36 peut, en variante, être constituée par un anneau gonflable, dont on peut faire varier le diamètre intérieur par gonflage.

Les modes de fonctionnement du dispositif 1 selon l'invention sont les suivants :
- dans le mode de respiration artificielle, le dispositif d'alimentation et de commande 22, d'une part, commande la vanne 29 à la fermeture par l'intermédiaire de la liaison 32, de sorte que le conduit 12 n'est pas alimenté en gaz et, d'autre part, adresse du gaz respirable dans le tube 4 par l'intermédiaire de la liaison 23. Ce dispositif 22 comporte des moyens (non représentés) pour permettre le réglage de la pression et du débit de gaz respirable qu'il reçoit de la conduite 26 et qu'il adresse au tube 4. Si une surpression se produit dans la voie respiratoire du patient, elle est détectée et transmise, par le canal supplémentaire 20 et la liaison 24, au dispositif 22 qui arrête son fonctionnement. De plus, si cette surpression dépasse le seuil de tarage de la soupape tarée 21 --par exemple du fait que le canal supplémentaire 20 est obstrué par des mucosités et n'a pu transmettre l'information de surpression au dispositif 22-- cette soupape 21 s'ouvre et le canal principal 5 est mis à l'atmosphère ;
- dans le mode d'assistance respiratoire, le dispositif d'alimentation et de commande 22 coupe la liaison 23 pour mettre l'orifice 6 en communication avec l'atmosphère et commande la vanne 29 par la liaison 32 pour que celle-ci adresse au patient des jets, continuels ou impulsionnels, de gaz respirable à travers la vanne 30, l'humidificateur 31 et les canaux auxiliaires 8.
   Il se forme alors, avec une pression de source 25 plus faible que dans le dispositif antérieur, la zone de pression 18 et la zone de dépression 19 permettant la ventilation aisée du patient. Celui-ci peut donc inspirer librement et profondément. Lorsqu'après une inspiration, le patient commence à expirer, le détecteur 33 détecte ce début d'expiration et commande la vanne 30 à la fermeture. Les jets de gaz respirables, la zone de pression 18 et la zone de dépression 19 disparaissent donc et le patient peut librement expirer à travers le canal principal 5.
   Si, pendant la ventilation, une surpression se produit dans la voie respiratoire du patient, comme cela a été décrit ci-dessus, cette surpression est détectée et transmise par le canal supplémentaire 20, de sorte que le dispositif 22 ferme la vanne 29 et que la ventilation est arrêtée.

De ce qui précède, on voit donc que, grâce à l'invention, on obtient, avec une source de pression modérée, une assistance respiratoire humidifiée efficace et sûre, avec disparition quasiment totale de l'espace mort inhérent aux sondes connues.

## Revendications

1. Dispositif d'assistance respiratoire comportant un tube (4) qui forme un canal principal (5) et qui est destiné à être relié par son extrémité distale (3) à une voie respiratoire d'un patient pour que ledit canal principal (5) relie, à l'extérieur, le système respiratoire dudit patient, ledit dispositif comportant de plus au moins un canal auxiliaire (8) qui est relié, à son extrémité proximale, à une source de gaz respirable (25) pour pouvoir insuffler un jet d'un tel gaz respirable dans ledit système respiratoire et dont l'extrémité distale débouche dans ledit canal principal (5) au voisinage de l'extrémité distale (7) de ce dernier, des moyens (14b) de déflexion dudit jet de gaz respirable de ventilation en direction de l'intérieur dudit canal principal (5) étant prévus en regard de l'orifice distal (17) dudit canal auxiliaire (8), de sorte que, en aval desdits moyens de déflexion, il se forme, à l'intérieur dudit canal principal, une zone de pression (18) de forme oblongue prenant naissance audit orifice distal (17) et s'allongeant dans la direction distale le long de l'axe (16) dudit canal principal (5) avec réduction progressive de sa section transversale en s'écartant de la paroi interne (15) dudit canal principal pour n'occuper que la partie centrale de ce dernier,
**caractérisé en ce qu'**il comporte une bague (36) disposée dans ledit canal principal (5) en aval desdits moyens de déflexion (14b) et **en ce que** ladite bague (36) entoure ladite zone de pression oblongue (18) en obturant au moins partiellement l'espace périphérique (37) dudit canal principal compris entre ladite paroi interne (15) de celui-ci et ladite zone de pression oblongue (18).

2. Dispositif selon la revendication 1,
**caractérisé en ce que** la distance (d) séparant ladite bague (36) desdits moyens de déflexion (14b) est approximativement égale au diamètre de la partie distale dudit canal principal (5).

3. Dispositif selon l'une des revendications 1 ou 2,
**caractérisé en ce que** la distance (d) séparant ladite bague (36) desdits moyens de déflexion (14b) est réglable.

4. Dispositif selon l'une des revendications 1 à 3,
**caractérisé en ce que** le diamètre intérieur de ladite bague (36) est réglable.

5. Dispositif selon l'une quelconque des revendications 1 à 4,
**caractérisé en ce qu'**il comporte un humidificateur (31) dans la conduite (28) reliant la source de gaz respirable (25) audit canal auxiliaire (8).

6. Dispositif selon l'une quelconque des revendications 1 à 5,
**caractérisé en ce qu'**il comporte un jeu de bagues (36) interchangeables, lesdites bagues présentant un diamètre intérieur différent.

7. Dispositif selon l'une quelconque des revendications 1 à 6,
**caractérisé en ce qu'**il comporte une vanne commandable (30) montée dans la conduite (28) reliant la source de gaz respirable (25) audit canal auxiliaire (8) et **en ce que** ladite vanne (30) est commandée par un capteur (33) détectant l'expiration dudit patient.

8. Dispositif selon la revendication 7,
**caractérisé en ce que** ledit capteur (33) détecte l'expiration du patient en aval de ladite bague (36), c'est-à-dire du côté de celle-ci opposé auxdits moyens de déflexion (14b).

## Patentansprüche

1. Vorrichtung zur Atmungsunterstützung, welche enthält: ein Rohr (4), welches einen Hauptkanal (5) bildet und welches dazu bestimmt ist, an seinem distalen Ende (3) mit einem Atemweg eines Patienten verbunden zu werden, damit der besagte Hauptkanal (5) die Verbindung vom Atmungssystem des besagten Patienten nach außen herstellt, wobei die besagte Vorrichtung außerdem mindestens einen Hilfskanal (8) enthält, der an seinem proximalen Ende mit einer Quelle (25) für Atemgas verbunden ist, damit ein Strom eines solchen Atemgases in das besagte Atmungssystem eingeblasen werden kann, und dessen distales Ende in den besagten Hauptkanal (5) in der Nähe des distalen Endes (7) dieses Letzteren einmündet, wobei Mittel (14b) zum Ablenken des besagten Stromes an zur Beatmung dienendem Atemgas in Richtung zum Innern des besagten Hauptkanals (5) gegenüber der distalen Öffnung (17) des besagten Hilfskanals (8) dergestalt vorhanden sind, dass sich hinter den besagten Ablenkmitteln im Innern des besagten Hauptkanals eine Druckzone (18) länglicher Form ausbildet, die an der besagten distalen Öffnung (17) ihren Ausgang nimmt und sich in distaler Richtung längs der Achse (16) des besagten Hauptkanals (5) in die Länge streckt, wobei ihr Querschnitt progressiv abnimmt, wobei sie sich von der Innenwand (15) des besagten Hauptkanals entfernt, so dass sie nur den zentralen Teil dieses Letzteren einnimmt,
**dadurch gekennzeichnet, dass** sie einen Ring (36) enthält, der in dem besagten Hauptkanal (5) hinter den besagten Ablenkmitteln (14b) angeordnet ist, und dadurch, dass der besagte Ring (36) die besagte längliche Druckzone (18) umgibt, wobei er zumindest teilweise den umfänglichen Raum (37) des besagten Hauptkanals zwischen der besagten Innenwand (15) desselben und der besagten länglichen Druckzone (18) absperrt.

2. Vorrichtung nach Anspruch 1,
**dadurch gekennzeichnet, dass** der Abstand (d) zwischen dem besagten Ring (36) und den besagten Ablenkmitteln (14b) annähernd gleich dem Durchmesser des distalen Teils des besagten Hauptkanals (5) ist.

3. Vorrichtung nach einem der Ansprüche 1 oder 2,
**dadurch gekennzeichnet, dass** der Abstand (d) zwischen dem besagten Ring (36) und den besagten Ablenkmitteln (14b) einstellbar ist.

4. Vorrichtung nach einem der Ansprüche 1 bis 3,
**dadurch gekennzeichnet, dass** der Innendurchmesser des besagten Rings (36) einstellbar ist.

5. Vorrichtung nach einem der Ansprüche 1 bis 4,
**dadurch gekennzeichnet, dass** sie einen Befeuchter (31) in der Leitung (28) aufweist, welche die Quelle für die das Atemgas (25) mit dem Hilfskanal (8) verbindet.

6. Vorrichtung nach einem der Ansprüche 1 bis 5,
**dadurch gekennzeichnet, dass** sie einen Satz von austauschbaren Ringen (36) aufweist, wobei die besagten Ringe einen unterschiedlichen Innendurchmesser haben.

7. Vorrichtung nach einem der Ansprüche 1 bis 6,
**dadurch gekennzeichnet, dass** sie ein steuerbares Ventil (30) enthält, das in die Leitung (28) eingebaut ist, welche die Quelle für das Atemgas (25) mit dem besagten Hilfskanal (8) verbindet, und dadurch, dass das besagte Ventil (30) durch einen Sensor (33) gesteuert wird, der das Ausatmen des besagten Patienten erfasst.

8. Vorrichtung nach Anspruch 7,
**dadurch gekennzeichnet, dass** der besagte Sensor (33) das Ausatmen des Patienten hinter dem besagten Ring (36) feststellt, d.h. auf der Seite, die derjenigen der besagten Ablenkmittel (14b) gegenüber liegt.

## Claims

1. Device for respiratory assistance comprising a tube (4) which forms a main channel (5) and which is intended to be connected via its distal end (3) to the respiratory tract of a patient so that said main channel (5) connects the respiratory system of said patient to the outside, said device moreover comprising at least one auxiliary channel (8) connected at its proximal end to a source of respirable gas (25) so as to insufflate a jet of such a respirable gas into said respiratory system, and whose distal end opens into said main channel (5) in the vicinity of the distal end (7) of the latter, means (14b) for deflecting said jet of respirable ventilation gas in the direction of the inside of said main channel (5) being provided opposite the distal orifice (17) of said auxiliary channel (8) so that, downstream of said means of deflection, inside said main channel, a pressure zone (18) of oblong shape is formed starting at said distal orifice (17) and extending in the distal direction along the axis (16) of said main channel (5), with gradual reduction of its cross section as it moves away from the inner wall (15) of said main channel so as to occupy only the central part of the latter, **characterized in that** the device comprises a ring (36) arranged in said main channel (5) downstream of said means of deflection (14b), and **in that** said ring (36) encloses said oblong pressure zone (18) by at least partially closing off the peripheral space (37) of said main channel situated between said inner wall (15) thereof and said oblong pressure zone (18).

2. Device according to Claim 1, **characterized in that** the distance (d) separating said ring (36) from said means of deflection (14b) is approximately equal to the diameter of the distal part of said main channel (5).

3. Device according to either of Claims 1 and 2, **characterized in that** the distance (d) separating said ring (36) from said means of deflection (14b) is adjustable.

4. Device according to one of Claims 1 to 3, **characterized in that** the internal diameter of said ring (36) is adjustable.

5. Device according to any one of Claims 1 to 4, **characterized in that** it comprises a humidifier (31) in the conduit (28) connecting the source of respirable gas (25) to said auxiliary channel (8).

6. Device according to any one of Claims 1 to 5, **characterized in that** it comprises a set of interchangeable rings (36), said rings having different internal diameters.

7. Device according to any one of Claims 1 to 6, **characterized in that** it comprises a controllable valve (30) mounted in the conduit (28) connecting the source of respirable gas (25) to said auxiliary channel (8), and **in that** said valve (30) is controlled by a sensor (33) detecting said patient's exhalation.

8. Device according to Claim 7, **characterized in that** said sensor (33) detects the patient's exhalation downstream of said ring (36), that is to say in the direction away from said means of deflection (14b).
